# EUROPEAN PATENT APPLICATION

(11) **EP 4 772 148 A2**
(43) Date of publication of application: **08.07.2026**
(21) Application number: 26178785.7
(22) Date of filing: 23.03.2022
(51) Int. Cl.: A61F 2/48

(54) **FLUID CONTROL SYSTEM FOR AN IMPLANTABLE INFLATABLE DEVICE**

(30) Priority: 25.03.2021 US 202163200738 P; 22.03.2022 US 202217655952
(62) Divisional of application: 22716807.7
(71) Applicant: Boston Scientific Scimed, Inc., Maple Grove, MN 55311-1566 (US)
(72) Inventor: GILDEA, John, Kildare, W23F40T (IE); SMITH, Noel, Kilkenny, R95 HX88 (IE); MARCOS LARANGEIRA, Eduardo, Clonmel Tipperary, E91 W1R9 (IE); SINNOTT, Thomas, Enniscorthy Wexford (IE); WATSCHKE, Brian P., Minneapolis, Minnesota 55405 (US); NOLAN, Daragh, Waterford, P36 TC98 (IE); BORGOS, Natalie Ann, Roseville, Minnesota 55113 (US)
(74) Representative: Pfenning, Meinig & Partner mbB

(57) **Abstract**

An implantable fluid operated device may include a fluid reservoir configured to hold fluid, an inflatable member, and a pump assembly configured to transfer fluid between the fluid reservoir and the inflatable member. The pump assembly may include one or more fluid pumps and one or more valves. The one or more valves may be normally open valves, normally closed valves, or a combination thereof. One or more sensing devices may be positioned within fluid passageways of the fluid operated device. The electronic control system may control operation of the pump assembly based on fluid pressure measurements and/or fluid flow measurements received from the one or more sensing devices. Variable voltage can be applied to the control of the pump and/or the valves based on varying atmospheric conditions and the fluid pressure and/or flow measurements processed by the electronic control system.

## Description

### CROSS-REFERENCE TO RELATED APPLICATIONS

This application is a continuation of, and claims priority to, U.S. Nonprovisional Patent Application No. 17/655,952, filed on March 22, 2022, entitled "FLUID CONTROL SYSTEM FOR AN IMPLANTABLE INFLATABLE DEVICE", which claims priority to U.S. Provisional Patent Application No. 63/200,738, filed on March 25, 2021, entitled "FLUID CONTROL SYSTEM FOR AN IMPLANTABLE INFLATABLE DEVICE", the disclosures of which are incorporated by reference herein in their entirety.

This application also claims priority to U.S. Provisional Patent Application No. 63/200,738, filed on March 25, 2021, the disclosure of which is incorporated by reference herein in its entirety.

### TECHNICAL FIELD

This disclosure relates generally to bodily implants, and more specifically to bodily implants including a pump.

### BACKGROUND

Active implantable fluid operated devices often include one or more pumps that regulate a flow of fluid between different portions of the implantable device. One or more valves can be positioned within fluid passageways of the device to direct and control the flow of fluid so as to achieve inflation, deflation, pressurization, depressurization, activation, deactivation and the like of different fluid filled implant components of the device. In some implantable fluid operated devices, sensors can be used to monitor fluid pressure and/or fluid volume and/or fluid flow within fluid passageways of the device. Accurate monitoring of conditions within the device, including pressure monitoring and flow monitoring, may provide for improved control of device operation, improved diagnostics, and improved efficacy of the device. In addition, sensors could be used to monitor external conditions from the device, including acceleration, angle, barometric pressure and temperature, which facilitate the determination of operating modes of the device.

### SUMMARY

In a general aspect, an implantable fluid operated inflatable device includes a fluid reservoir; an inflatable member; and a fluid control system configured to transfer fluid between the fluid reservoir and the inflatable member. The fluid control system can include a housing; at least one valve and at least one pump positioned in a fluid passageway within in the housing; a first fluid port in fluidic communication with the fluid reservoir; and a second fluid port in fluidic communication with the inflatable member. The implantable fluid operated inflatable device can also include at least one pressure sensing device configured to sense a fluid pressure in the implantable fluid operated inflatable device; and an electronic control system configured to receive the pressure sensed by the at least one pressure sensing device, and to control the at least one valve and/or at least one pump in response to the received pressure.

In some implementations, the at least one valve and the at least one pump includes a combined pump and valve device positioned inline between the reservoir and the inflatable member, including a chamber; a diaphragm positioned along an edge portion of the chamber; a piezoelectric element mounted on the diaphragm; a first valve positioned at a first end portion of the chamber corresponding to a first end portion of the piezoelectric element; and a second valve positioned at a second end portion of the chamber corresponding to a second end portion of the piezoelectric element. In some implementations, in a first mode in which fluid is moved through the combined pump and valve device in a first direction to transfer fluid from the reservoir to the inflatable member to inflate the inflatable member, a first pumping cycle of the combined pump and valve device can include a first supply stroke in which fluid is drawn into the chamber through the first valve while the second valve is closed; and a first pressure stroke in which fluid is expelled out of the chamber through the second valve while the first valve is closed. In a second mode in which fluid is moved through the combined pump and valve device in a second direction to transfer fluid from the inflatable member to the reservoir to deflate the inflatable member, a second pumping cycle of the combined pump and valve device can include a second supply stroke in which fluid is drawn into the chamber through the second valve while the first valve is closed; and a second pressure stroke in which fluid is expelled out of the chamber through the first valve while the second valve is closed. In some implementations, the first supply stroke and the first pressure stroke are alternately and repeatedly implemented until an inflation pressure of the inflatable member is achieved based on a pressure or other characteristics sensed by the at least one sensing device; and the second supply stroke and the second pressure stroke are alternately and repeatedly implemented until a deflation pressure is achieved based on a pressure sensed by the at least one sensing device.

In some implementations, the at least one valve is a piezoelectric valve, including a valve base or host; at least one inlet port formed in the valve base or host; at least one outlet port formed in the valve base or host; a diaphragm coupled to the valve base or host; and a piezoelectric element mounted on the diaphragm, wherein a voltage applied to the piezoelectric element is a variable voltage to maintain a set state of the fluid operated inflatable device based on a pressure detected in the fluid passageway of the valve relative to a detected pressure external to the valve. The variable voltage applied to the piezoelectric element to maintain the set state of the fluid operated inflatable device may be based on the pressure detected in the fluid passageway of the valve relative to an atmospheric pressure sensed by the electronic control system. The variable voltage applied to the piezoelectric element may adjust a position of the piezoelectric element and the diaphragm so as to adjust at least one of a fluid pressure or a fluid flow rate to adjust for atmospheric conditions and correspond to the set state of the fluid-controlled inflatable device. The voltage applied to the piezoelectric element may be selected from a calibration curve associated with the piezoelectric valve that is accessible in a memory of the electronic control system.

In some implementations, the at least one valve is a normally open piezoelectric valve that is configured to transition from a normally open state to a closed state in response to an application of voltage to the piezoelectric element, and to return to the normally open state in response to release of the voltage. The normally open piezoelectric valve may be configured to remain in the closed state for a period of time after release of the voltage, and to transition to the normally open state in response to dissipation of electrical bias accumulated in the piezoelectric element. In some implementations, the normally open piezoelectric valve includes a resistor electrically connected to the piezoelectric element. The resistor may be configured to control a dissipation of electrical bias accumulated in the piezoelectric element such that the normally open piezoelectric valve transitions from the closed state to the normally open state in a set period of time after release of the voltage.

In some implementations, the piezoelectric valve is a normally closed piezoelectric valve that is configured to transition from a normally closed state to an open state in response to an application of voltage to the piezoelectric element, and to return to the normally closed state in response to release of the voltage. The normally closed piezoelectric valve may include a plunger movably positioned within the fluid passageway of the normally closed piezoelectric valve, wherein the plunger is sealed against the valve base in the normally closed state so as to restrict flow through the fluid passageway, and is spaced apart from the valve base in the open state so as to open the fluid passageway. In the normally closed state of the normally closed piezoelectric valve, a backpressure applied to the plunger through the at least one outlet maintains the sealed position of the plunger against the valve base in response to a surge in fluid pressure at the at least one inlet.

In some implementations, the electronic control system includes a printed circuit board including a memory configured to store at least one control algorithm, a communication module configured to communicate with one or more external devices, and a processor configured to receive pressure level measurements from the at least one sensing device; apply the at least one control algorithm based on the received pressure level measurements; and control operation of the at least one valve and the at least one pump in accordance with the applied at least one control algorithm.

In some implementations, the implantable fluid operated inflatable device is an artificial urinary sphincter or an inflatable penile prosthesis.

In another general aspect, a method of controlling an implantable fluid operated inflatable device includes receiving, by a processor of the inflatable device from a pressure sensing device within a fluid passageway of the inflatable device, a fluid pressure measurement; comparing, by the processor, the measured pressure received from the pressure sensing device to a pressure external to the fluid passageway; and controlling, by the processor, a circuit to apply a voltage to a piezoelectric element of a piezoelectric valve of the inflatable device based on the comparison to maintain a set condition of the inflatable device.

In some implementations, controlling the circuit to apply the voltage to the piezoelectric element includes detecting, based on the comparison, a change in atmospheric pressure from a calibration condition of the inflatable device based on the comparison; selecting, by the processor, a voltage to be applied to a piezoelectric element of a piezoelectric valve of the inflatable device from a previously stored lookup table in response to the detected change in atmospheric pressure; and applying to the selected voltage to the piezoelectric element to maintain a set condition of the inflatable device in the changed atmospheric conditions.

In some implementations, the piezoelectric valve is a normally open piezoelectric valve, and wherein controlling the circuit to apply the voltage to the piezoelectric element includes controlling a resistor in the circuit such that electrical bias accumulated in the piezoelectric element dissipates over a set period of time to return the normally open piezoelectric valve to a normally open state.

In some implementations, the piezoelectric valve is a normally closed piezoelectric valve, and the method also includes detecting a surge in fluid pressure at an inlet portion of the piezoelectric valve; and applying a backpressure at an outlet portion of the piezoelectric valve in response to the surge in fluid pressure at the inlet portion to maintain a closed state of the normally closed piezoelectric valve.

In some implementations, the method also includes receiving, by a control module of the processor, a user input from an external device in communication with the processor; and adjusting at leasta one of a fluid pressure or a fluid flow rate in the inflatable device in response to the received user input.

### BRIEF DESCRIPTION OF THE DRAWINGS

FIG. 1 is a block diagram of an implantable fluid operated device according to an aspect.
FIGS. 2A and 2B illustrate example implantable fluid operated devices according to an aspect.
FIGS. 3A and 3B are schematic diagrams of a fluid architecture of an implantable fluid operated device according to an aspect.
FIGS. 4A and 4B illustrate an open state and a closed state, respectively, of a normally open piezoelectric valve according to an aspect.
FIGS. 5A-5C illustrate a fully open state, a partially open state, and a closed state of a piezoelectric valve according to an aspect.
FIGS. 6A-6C illustrate operation of a normally open valve according to an aspect.
FIG. 7 is a graph of variable closing voltage curves.
FIGS. 8A-8C illustrate operation of an example valve according to an aspect.
FIG. 9 is a schematic diagram of a fluid architecture of an implantable fluid operated device including a valve.
FIGS. 10A-10D illustrate operation of an example pump and valve device according to an aspect.

### DETAILED DESCRIPTION

Detailed implementations are disclosed herein. However, it is understood that the disclosed implementations are merely examples, which may be embodied in various forms. Therefore, specific structural and functional details disclosed herein are not to be interpreted as limiting, but merely as a basis for the claims and as a representative basis for teaching one skilled in the art to variously employ the implementations in virtually any appropriately detailed structure. Further, the terms and phrases used herein are not intended to be limiting, but to provide an understandable description of the present disclosure.

The terms "a" or "an," as used herein, are defined as one or more than one. The term "another," as used herein, is defined as at least a second or more. The terms "including" and/or "having", as used herein, are defined as comprising (i.e., open transition). The term "coupled" or "moveably coupled," as used herein, is defined as connected, although not necessarily directly and mechanically.

In general, the implementations are directed to bodily implants. The term patient or user may hereinafter be used for a person who benefits from the medical device or the methods disclosed in the present disclosure. For example, the patient can be a person whose body is implanted with the medical device or the method disclosed for operating the medical device by the present disclosure.

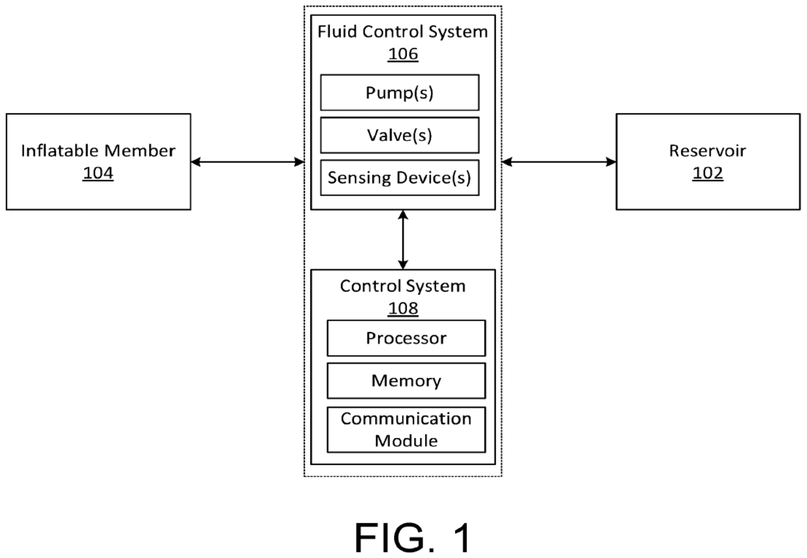

FIG. 1 is a block diagram of an example implantable fluid operated inflatable device 100. The example device 100 shown in FIG. 1 includes a fluid reservoir 102, an inflatable member 104, and a fluid control system 106 including fluidics components such as one or more pumps, one or more valves and the like configured to transfer fluid between the fluid reservoir 102 and the inflatable member 104. The fluid control system 106 can include on or more sensing devices that sense conditions such as, for example, fluid pressure, fluid flow rate and the like within the fluidics system of the device 100. In some implementations, the example device 100 includes an electronic control system 108. The electronic control system 108 may provide for the monitoring and/or control of the operation of various fluidics components of the fluid control system 106 and/or communication with one or more sensing device(s) within the implantable fluid operated inflatable device 100 and/or communication with one or more external device(s). In some examples, the electronic control system 108 includes, for example, a processor, a memory, a communication module, and other such components configured to provide for the operation and control of the implantable fluid operated inflatable device 100. For example, the communication module may provide for communication with one or more external devices. The one or more external devices may be configured to receive user inputs and transmit the user inputs to the electronic control system 108 for processing, operation and control of the device 100. The electronic control system 108 may, through the communication module, transmit operational information to the external device for user consumption. The fluid reservoir 102, the inflatable member 104, and the fluid control system 106 may be internally implanted into the body of the patient. In some implementations, the electronic control system 108 is coupled to or incorporated into a housing of the fluid control system 106. In some implementations, at least a portion of the electronic control system 108 is physically separate from the fluid control system 106. In some implementations, some modules of the electronic control system 108 are coupled to or incorporated into the fluid control system 106, and some modules of the electronic control system 108 are separate from the fluid control system 106. For example, in some implementations, some modules of the electronic control system 108 are included in an external device that is in communication other modules of the electronic control system 108 included within the implantable device 100. In some implementations, operation of the implantable fluid operated inflatable device 100 may be manually controlled.

In some examples, electronic monitoring and control of the fluid operated device 100 may provide for improved patient control of the device, improved patient comfort, and improved patient safety. In some examples, electronic monitoring and control of the fluid operated device 100 may afford the opportunity for tailoring of the operation of the device 100 by the physician without further surgical intervention.

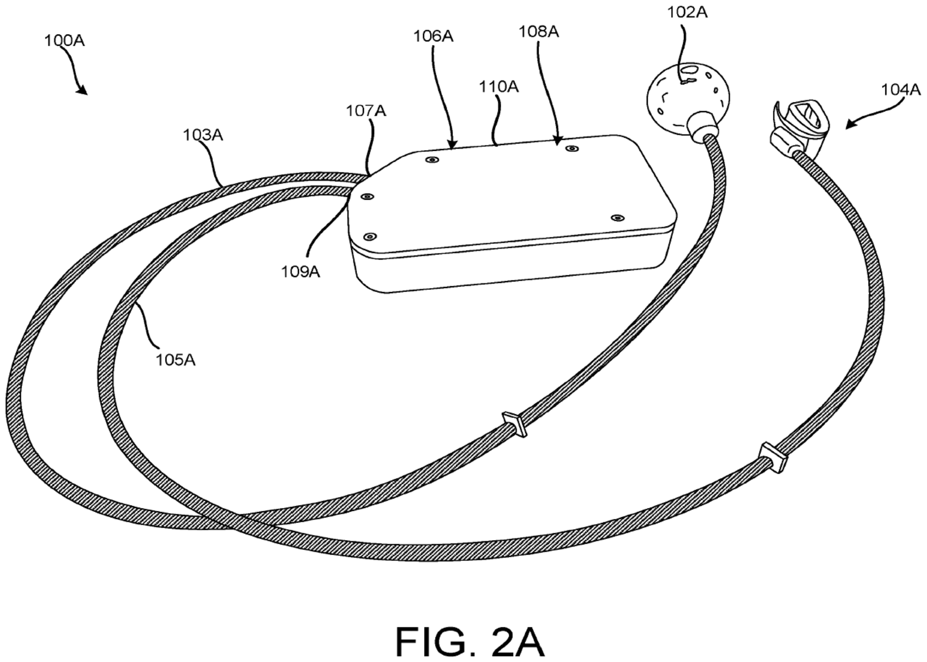

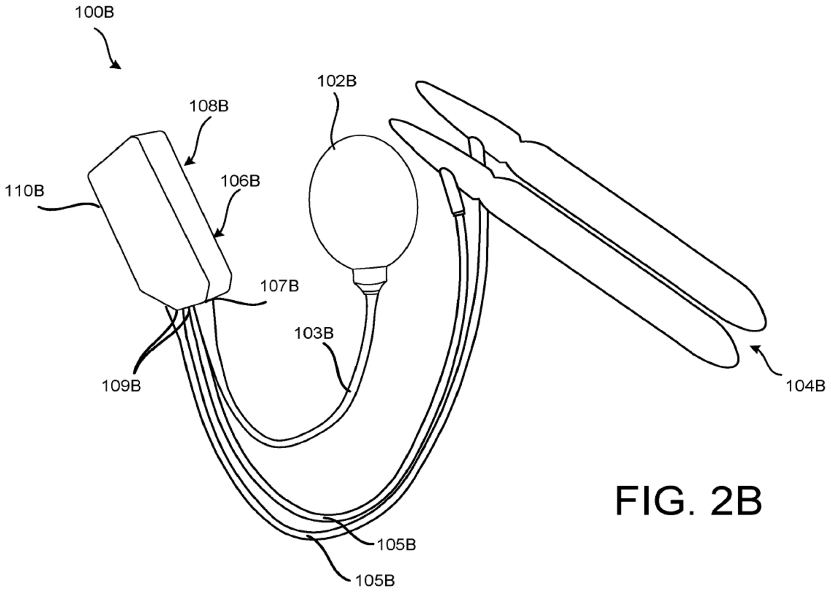

The example implantable fluid operated device 100 may be representative of a number of different types of implantable fluid operated devices. For example, the device 100 shown in FIG. 1 may be representative of an artificial urinary sphincter 100A as shown in FIG. 2A. The example artificial urinary sphincter 100A shown in FIG. 2A includes a fluid control system 106A including fluidics components such as pumps, valves and the like positioned in fluid passageways, and an electronic control system 108A configured to provide for the transfer of fluid between a reservoir 102A and an inflatable cuff 104A. Fluidics components of the fluid control system 106A, and electronic components of the electronic control system 108A may be received in a housing 110A. A first conduit 103A connects a first fluid port 107A of the fluid control system 106A/electronic control system 108A received in the housing 110A with the reservoir 102A. A second conduit 105A connects a second fluid port 109A of the fluid control system 106A/electronic control system 108A received in the housing 110A with the inflatable cuff 104A. In some examples, the device 100 shown in FIG. 1 may be representative of an inflatable penile prosthesis 100B as shown in FIG. 2B. The example penile prosthesis 100B shown in FIG. 2B includes a fluid control system 106B including fluidics components such as pumps, valves and the like positioned in fluid passageways, and an electronic control system 108B configured to provide for the transfer of fluid between a fluid reservoir 102B and inflatable cylinders 104B. Fluidics components of the fluid control system 106B, and electronic components of the electronic control system 108B may be received in a housing 110B. A first conduit 103B connects a first fluid port 107B of the fluid control system 106B/electronic control system 108B received in the housing 110B with the reservoir 102B. One or more second conduits 105B connect one or more second fluid ports 109B of the fluid control system 106A/electronic control system 108A received in the housing with the inflatable cylinders 104B. The principles to be described herein may be applied to these and other types of implantable fluid operated inflatable devices that rely on a pump assembly including various fluidics components to provide for the transfer of fluid between the different fluid filled implantable components to achieve inflation, deflation, pressurization, depressurization, deactivation and the like for effective operation. The example devices 100A, 100B shown in FIGS. 2A and 2B include electronic control systems 108A, 108B to provide for the monitoring and control of pressure and/or fluid flow through the respective devices 100A, 100B. Some of the principles to be described herein may also be applied to implantable fluid operated inflatable devices that are manually controlled.

As noted above with respect to FIG. 1, the fluid control system 106 can include a pump assembly including, for example, one or more pumps and one or more valves positioned within a fluid circuit of the pump assembly to control the transfer fluid between the fluid reservoir and the inflatable member. In some examples, the pump(s) and/or the valve(s) are electronically controlled. In some examples, the pump(s) and/or the valve(s) are manually controlled. In some examples, the pump assembly includes a fluid manifold having fluidic channels formed therein, defining the fluid circuit. In an example in which the pump assembly is electronically powered and/or controlled, the manifold may be a hermetic manifold that can contain and segment the flow of fluid from electronic components of the pump assembly, to prevent leakage and/or gas exchange. In some examples, the pump assembly includes one or more pressure sensing devices in the fluid circuit to provide for relatively precise monitoring and control of fluid flow and/or fluid pressure within the fluid circuit and/or the inflatable member. A fluid circuit configured in this manner may facilitate the proper inflation, deflation, pressurization, depressurization and deactivation of the components of the implantable fluid operated device to provide for patient safety and device efficacy.

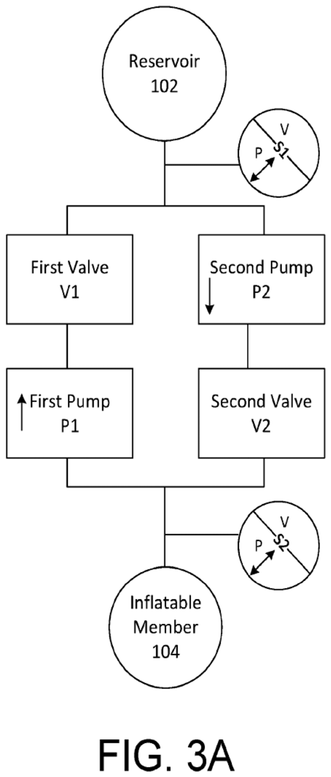

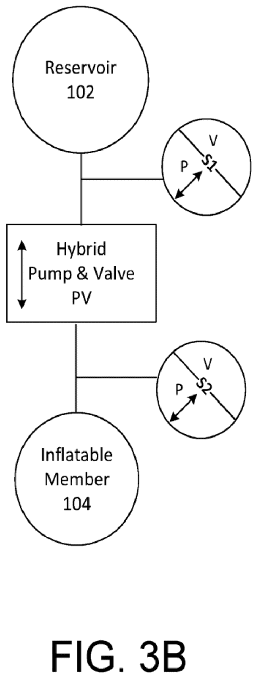

FIGS. 3A and 3B are a schematic diagrams of example fluidic architectures for an implantable fluid operated device, according to an aspect. The fluidic architecture of an implantable fluid operated device can include other orientations of fluidic channels, valve(s), pressure sensor(s) and other components than shown in FIGS. 3A and 3B. A fluidic architecture that can accommodate back pressure, pressure surges and the like enhances the performance, efficacy and efficiency of the fluid operated device 100.

The example fluidic architecture shown in FIG. 3A includes channels guiding the flow of fluid between the reservoir 102 and the inflatable member 104. In the example shown in FIG. 3A, a first valve V1 in a first fluidic channel controls the flow of fluid, generated by a first pumping device P1, from the inflatable member 104 to the reservoir 102. A second valve V2 in a second fluidic channel controls the flow of fluid, generated by a second pumping device P2, from the reservoir 102 to the inflatable member 104. In the example shown in FIG. 3A, a first pressure sensing device S1 senses a fluid pressure at the reservoir 102, and a second pressure sensing device S2 senses a fluid pressure at the inflatable member 104. The first and second pressure sensing devices S1, S2 may provide for the monitoring of fluid flow and/or fluid pressure in the fluidic channels. In the arrangement shown in FIG. 3A, one of the first pump P1 or the second pump P2 is active, while the other of the first pump P1 or the second pump P2 is in a standby mode, such that the first and second pumps do not typically operate simultaneously. For example, operation of the first pump P1 (with the second pump P2 in the standby mode) may provide for the deflation of the inflatable member 104, and operation of the second pump P2 (with the first pump P1 in the standby mode) may provide for the inflation of the inflatable member 104. The valves V1, V2 may provide for the selective sealing of the respective fluidic channel(s) so as to maintain a set state of the fluid operated device. For example, selective sealing of the respective fluidic channel(s) by the valves V1, V2 may maintain an inflated state or a deflated state of the inflatable member 104. Interaction with the valves V1, V2 (and the corresponding change in fluid flow through the fluidic architecture of the device) may change the set state of the fluid operated device. Valves V1, V2 that maintain the set state of the device until the patient requires a change in the set state of the device and initiates the required change in the set state of the device provide enhanced patient safety and improved device efficacy.

In some examples, a valve and a pump can be incorporated into a single component that provides for both the generation of fluid flow, and the control of fluid flow in the fluid operated device. In some examples, the pump can be a multi-directional pump that can pump fluid in multiple directions. The example fluidic architecture shown in FIG. 3B includes a hybrid pump and valve device PV, or a combined pump and valve device PV, including a multi-directional pumping device and one or more valves that control fluid flow through the combined pump and valve device PV. The pump and valve device PV can pump fluid in a first direction (for example, from the reservoir 102 toward the pump and valve device PV, and the pump and valve device PV toward the inflatable member 104) and in a second direction (for example, from the inflatable member 104 toward the pump and valve device PV, and the pump and valve device PV toward the reservoir 102). The incorporation of the hybrid, or combined pump and valve device PV may provide for fluid pumping and flow control in the fluid operated device using fewer components in the fluidic architecture. In some examples, this may reduce the overall size of the fluid control system including the pump assembly, any may reduce power consumption, thus increasing longevity of a power storage device, or battery, of the fluid operated device 100.

In some examples, the pump(s) P1, P2 and valve(s) V1, V2 (and/or the hybrid, or combined, pump and valve device PV) included in the fluidic architecture can each include chambers that are actuated on by a diaphragm. For example, a pump can include a first check valve at an inlet port of the pump chamber and a second check valve at an outlet port of the pump chamber, such that oscillation of the diaphragm causes forward flow (i.e. flow from the inlet port toward the outlet port). Similarly, a valve can include a valve chamber, with actuation of the diaphragm causing closure of a flow path between inlet and outlet ports of the valve chamber.

In some examples, a multi-directional pump and valve device can include a vibrating or oscillating diaphragm is positioned between a first port and a second port of a fluid chamber, with a first electronically controlled valve at the first port and a second electronically controlled valve at the second port. The first and second valves may be opened and closed in a sequence that provides for pumping in a first direction (i.e., from the first port toward the second port), for pumping in a second direction (i.e., from the second port toward the first port), or for closure of the fluid flow path. In some examples, two vibrating or oscillating diaphragms may be placed in series in a fluid flow path, for example, side to side or face to face in the fluid flow path. The first and second oscillating diaphragms act as diffusers in the flow path that sequentially restrict the flow of fluid through the flow path, in a first direction and then in a second direction. The sequentially alternating restriction of flow through the flow path in the first and second directions cause flow in either the first direction or the second direction, depending on the pattern produced by the oscillation of the first and second diaphragms and the alternating restriction of flow.

In some examples, the valves included in the fluidic architecture of a fluid operated device may be normally open valves. The use of normally open valves may provide a failsafe measure in the operation of the types of fluid operated devices as described above, particularly in a situation in which the fluid operated device is electronically controlled. For example, a failure of one of the valves within the fluid operated device, and/or an overall device failure which results in an inability to change states (for example, a sustained inflated state of the inflatable member and/or an inability to transition from an inflated state to a deflated state) may cause patient discomfort and may compromise patient safety. The use of a normally open valve and the corresponding normally open state of the valve would allow for release of pressure (for example, deflation of the inflatable member 104) and allow the fluid within the device to reach an equilibrium state, to provide for patient comfort and safety.

In some examples, one or more of the valves included in the fluidic architecture are piezoelectric valves and, in some examples, normally open piezoelectric valves. Piezoelectric materials produce electrical energy when subjected to mechanical deformation of strain. Conversely, piezoelectric materials are deformed in response to application of an electrical field. These properties allow mechanical valves to be electronically controlled through the application of voltage to the valves.

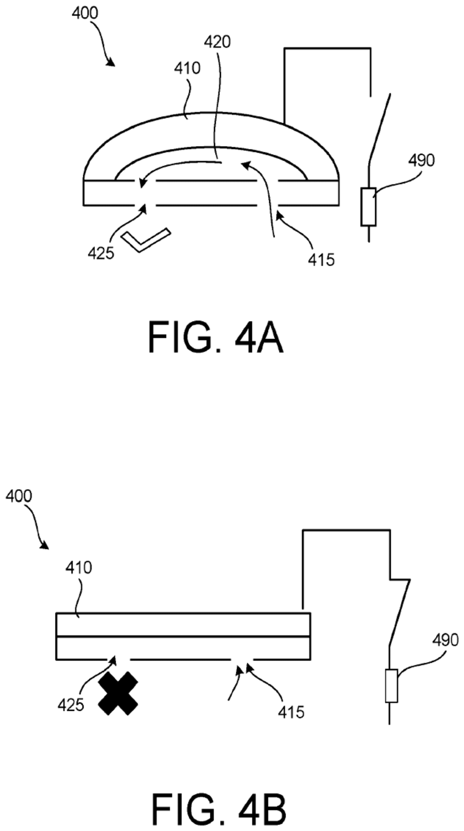

In a normally open piezoelectric valve, the valve is in the open position in the passive, or equilibrium state. The normally open valve closes in response to an application of voltage. FIG. 4A illustrates an example of normally open piezoelectric valve 400 in the equilibrium state, in which the valve 400 is open. FIG. 4B illustrates the normally open piezoelectric valve 400 in the closed state. The example normally open piezoelectric valve 400 shown in FIGS. 4A and 4B includes a piezoelectric element 410 coupled to a valve base 450. Fluid ports 415, 425 are formed in the base 450. In the example shown in FIGS. 4A and 4B, the first fluid port 415 is an inlet port 415, and the second fluid port 425 is an outlet port. A fluid chamber 420 is defined in a space between the piezoelectric element 410 and the valve base 450.

As shown in FIG. 4A, in the equilibrium state of the normally open piezoelectric valve 400 (i.e., the open state), no voltage is applied to the piezoelectric element 410 of the valve 400. In the equilibrium state in which the valve 400 is open, the piezoelectric element 410 of the valve 400 is deformed or deflected, allowing fluid to flow into the chamber 420 through the first port 415, and out of the chamber 420 through the second port 425. Application of a voltage to the piezoelectric element 410 of the valve 400 causes the valve 400 to close, as shown in FIG. 4B. In some examples, an electrical bias may remain in the piezoelectric element 410 after removal of the voltage. The electrical bias accumulated in the piezoelectric element 410 of the valve may dissipate over time, maintaining the closed state of the valve 400 through at least a portion of the dissipation period. In some examples, a resistor 490 may be positioned in the circuit, in parallel to the piezoelectric element 410. The resistor 490 may provide for the controlled dissipation of voltage accumulated in the piezoelectric element 410, so that the normally open valve 400 is returned to the equilibrium/open state shown in FIG. 4A in a time-controlled manner.

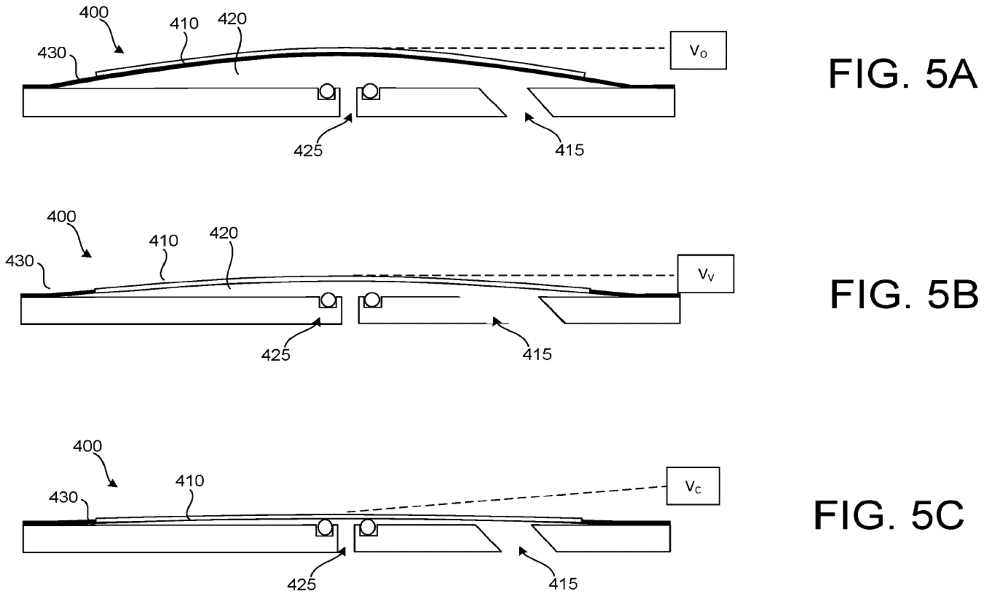

In some examples, a voltage level applied to the valve 400 may be varied, to, for example, vary an opening amount of the valve 400. vary a flow rate through the valve 400 and the like. In some examples, variable voltage control may be applied to account for changes in atmospheric pressure (for example, changes in altitude or depth) which affect pressure of fluid flowing in the fluid operated device, and thus can affect the proper operation of the fluid operated device. FIG. 5A illustrates a state of the valve 400 in which a fully open voltage Vo is applied to the piezoelectric element 410 of the valve 400 such that the valve 400 is in a fully open state. FIG. 5B illustrates a state of the valve 400 in which a partially open voltage Vv, or variable voltage Vv is applied to the valve 400 such that the valve 400 is in a partially open state. FIG. 5C illustrates a state of the valve 400 in which a fully closed voltage Vc is applied to the valve 400 such that the valve 400 is in a fully closed state. In a case in which the valve 400 is the normally open piezoelectric valve 400 described above, the normal, equilibrium state is the open state shown in FIG. 5A, and thus the fully open voltage Vo would be essentially zero.

In some examples, as the fluid operated device experiences changes in atmospheric and/or working pressure (due to, for example, changes in altitude and/or depth), a corresponding change in fluid pressure and/or fluid flow rate through the valve 400 may be experienced. Without adjustment, these changes in fluid pressure and/or fluid flow rate may impact (adversely impact) the proper operation of the fluid operated device.

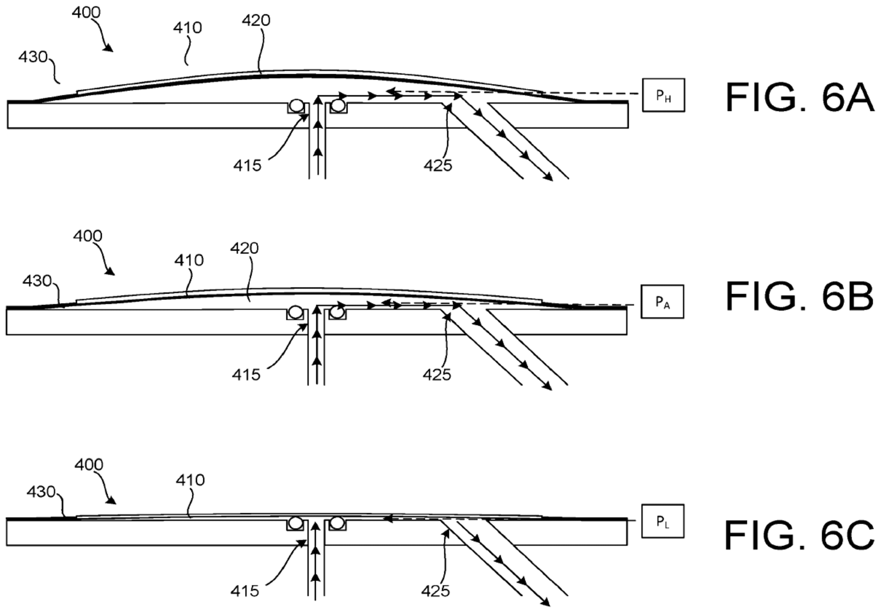

FIG. 6A illustrates the piezoelectric valve 400 in a state in which a pressure P_{H} in the chamber 420 and fluid passageways is relatively high compared to the pressure outside of the device (i.e., atmospheric and/or working pressure), thus drawing the piezoelectric element 410 and diaphragm 430 away from the chamber 420 and increasing a flow rate through the valve 400. FIG. 6B illustrates the piezoelectric valve 400 in a state in which the pressure P_{A} in the chamber 420 and fluid passageways is essentially the same as outside of the device. FIG. 6C illustrates the piezoelectric valve 400 in a state in which the pressure P_{L} in the chamber 420 and fluid passageways is measurably less than outside of the device, thus pulling the piezoelectric element 410 and diaphragm 430 toward the chamber 420 close off the fluid passageways and restrict fluid flow through the valve 400.

As noted above, the fluid operated device may experience varying levels of atmospheric and/or working pressure, which changes with, for example, altitude or depth. For example, atmospheric and/or working pressure decreases as altitude increases, and thus a patient having an implanted fluid operated device may experience decreased atmospheric and/or working pressure when flying. The decreased atmospheric and/or working pressure may affect operation of the piezoelectric valve 400 as shown in FIG. 6A. That is, the decreased atmospheric and/or working pressure may cause the baseline position of the diaphragm 430 to change as shown, causing an increased fluid flow rate through the valve 400. A patient having an implanted fluid operated device may experience increased atmospheric and/or working pressure when, for example, submerged or swimming in water. The increased atmospheric and/or working pressure may affect operation of the piezoelectric valve 400 as shown in FIG. 6C. That is, the increased atmospheric and/or working pressure may cause the baseline position of the diaphragm 430 to change as shown, causing a decrease in fluid flow rate through the valve 400, or restricting flow through the valve 400. Without correction/recalibration for the movement of the diaphragm 430 in response to the variation in atmospheric and/or working pressure, either of these situations may result in improper operation of the fluid operated device, to the point where patient comfort and safety could be impacted.

An electronically controlled fluid operated device has the ability to obtain atmospheric and/or working pressure substantially real time. The electronically controlled fluid operated device may use detected atmospheric and/or working pressure levels, and detected changes in atmospheric and/or working pressure levels, to adjust pressure levels in the fluid operated device, and in particular to adjust operation of flow control valves in the fluid operated device (for example, open/close level of the valves), to ensure proper fluid pressure levels and fluid flow rates for safe operation of the fluid operated device.

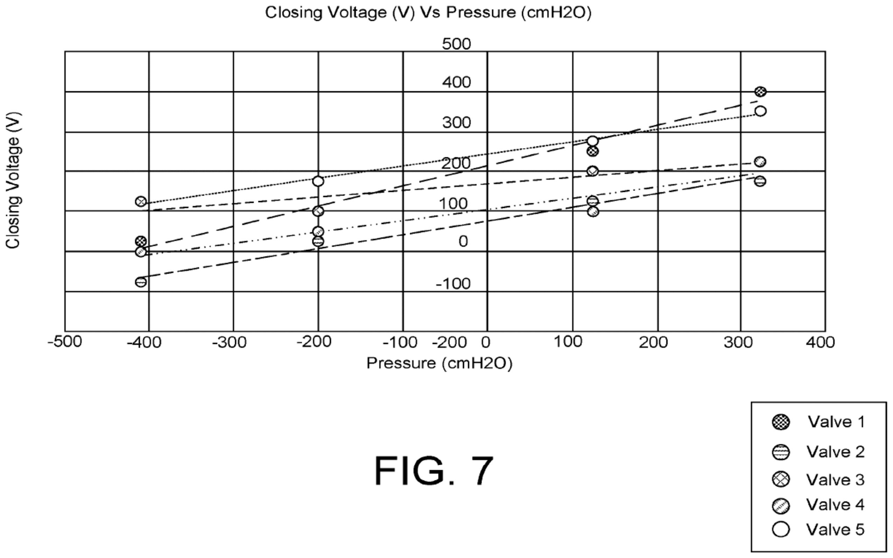

In some examples, a variable closing voltage may be applied to one or more of the valves 400 in the fluid operated device to provide a corrected level of closure of the valve 400 corresponding to the varying levels of atmospheric and/or working pressure the valve 400 may experience, based on for example, altitude or depth. In some examples, a calibration curve may be referenced to determine an appropriate closing voltage for a given atmospheric and/or working pressure sensed by the electronically controlled fluid operated device. An example calibration curve is shown in FIG. 7. The example calibration curve shown in FIG. 7 illustrates the voltage required to close a fluid passageway at different atmospheric and/or working pressures for a particular valve.

In some examples, a calibration curve for each valve 400 of the fluid operated device can be stored, for example, in the form of a look up table, in a memory of the electronic control system 108. During operation, as atmospheric and/or working pressure (and changes in atmospheric and/or working pressure) is sensed, the electronic control system 108 can access the appropriate calibration curve/look up table for each of the valves 400, and adjust a voltage level applied to each of the valves 400 accordingly, to maintain a current state of the fluid operated device.

The ability to determine variable closing voltages for each of the flow control valves within the fluid operated device to account for varying atmospheric conditions (for example, as pressure varies with altitude and/or depth) and to adjust applied voltages accordingly to maintain a current state of the fluid operated device may provide for the proper operation of the fluid operated device even when subjected to varying atmospheric conditions. This may improve patient comfort and safety considerations. The risk of over-driving the piezoelectric element, and adversely impacting overall device performance, may be greatly reduced, particularly in a high altitude (low atmospheric and/or working pressure) situation, in which the pressure in the fluid passageways is relatively low. The ability to apply an adjusted closing voltage (for example, a lower closing voltage) may improve reliability of the fluid operated device by reducing or substantially eliminating leakage issues in response to increased pressure levels in the fluid passageways at depths and reduced pressure levels in the fluid passageways at altitudes. Improved, and even optimum, sealing may be achieved by applying an adjusted voltage to the valve 400 at a particular atmospheric condition, thus providing for the proper fluid flow volume and rate through the valve 400 and avoiding damage to the valve 400. The proper sealing of the fluid passageways at varying atmospheric conditions afforded by the use of the calibration curves to determine proper closing voltages for the valves 400 may guard against over-pressuring of the inflatable member, reduce risk of piezo damage thus further enhancing patient safety and comfort.

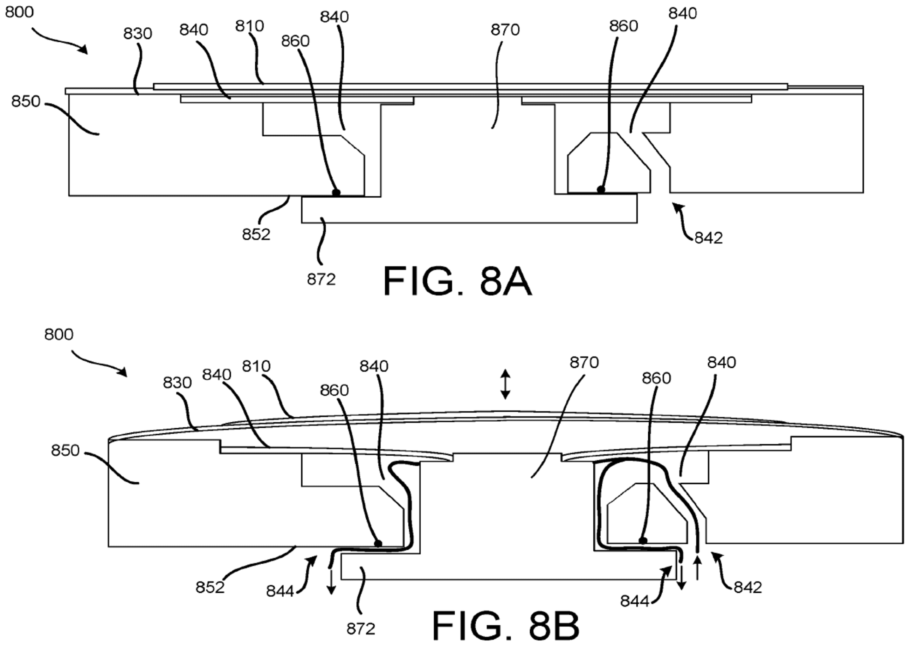

In some examples, the fluidic architecture of a fluid operated device may include one or more normally closed valves. A normally closed valve may provide for maximum sealing without the need for activation (such as, for example the application of voltage), which may be advantageous in some positions within the fluid operated device, and in some situations. FIG. 8A illustrates an example normally closed valve 800 in closed state. FIG. 8B illustrates the example normally closed valve 800 in an open state.

The example normally closed valve 800 shown in FIGS. 8A and 8B includes a plunger 870 movably positioned with respect to a valve base 850 so as to selectively block a fluid passageway 880 defined in the valve base 850. In the closed state shown in FIG. 8A, a flange 872 of the plunger 870 is positioned against a sealing surface 852 of the valve base 850, with an O-ring 860 positioned in a sealing notch formed in the valve base 850. In some examples, the O-ring may be positioned in a sealing notch formed in the flange 872 of the plunger 870. A piezoelectric element 810 is mounted on an external foil 830 coupled to the valve base 850. An internal foil 840 is fixed to the plunger 870 and to the base 850.

In the closed state of the valve 800 shown in FIG. 8A, the piezoelectric element 810 has not been actuated (i.e., a voltage has not been applied), and the flange 872 of the plunger 870 is positioned against the sealing surface 852 of the valve base 850, thus forming a seal that blocks the fluid passageway 880. To change the state of the normally closed valve 800 from the closed state to the open state shown in FIG. 8B, a voltage is applied to the piezoelectric element 810, causing a deflection of the piezoelectric element 810, the external foil 830, and the internal foil 840. In particular, the application of voltage to the piezoelectric element 810 has caused an upward (in the example orientation shown in FIG. 8B) of the piezoelectric element 810 mounted on the external foil 830, and a downward deflection of the internal foil 840 attached to the valve base 850 and the plunger 870.This downward deflection of the internal foil 840 drives the plunger 870 downward together with the internal foil 840. Deflection of the piezoelectric element 810 and the external and internal foils 830, 840 and movement of the plunger 870 in this manner allows fluid to flow through the valve 800 as shown in FIG. 8B. That is, in the open state shown in FIG. 8B, the plunger 870 has moved downward (in the example orientation shown in FIGS. 8A and 8B), away from the valve base 850, such that a space is formed between the flange 872 of the plunger 870 and the sealing surface 852 of the of the valve base 850. This movement releases the seal between the plunger 870 and the valve base 850, allowing fluid to flow into the fluid passageway 880 through at least one inlet 842, and out of the fluid passageway 880 through one or more outlets 844. In some examples, cyclic application and release of voltage applied to the piezoelectric element 810 may generate reciprocal movement of the plunger 870 as the plunger 870 alternates between the open position shown in FIG. 8A and the closed position shown in FIG. 8B.

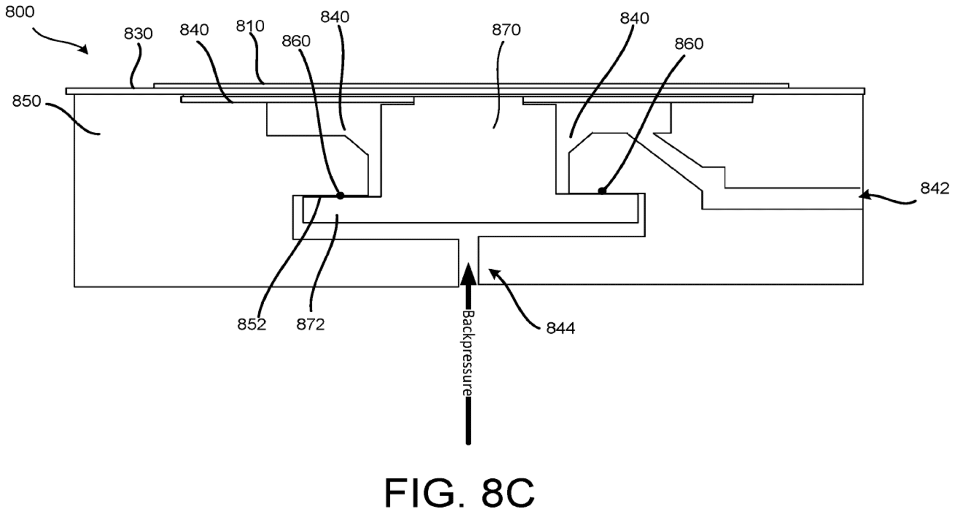

In some situations, the normally closed valve 800 may experience a sudden surge in pressure of the fluid due to, for example, a fall, physical exertion and the like. The sudden surge or spike in pressure may cause the plunger 870 to move, resulting in leakage through the valve 800 as the valve 800 is forced from the closed position to the open position. Application of a back pressure at the outlet 844 of the normally closed valve 800 as shown in FIG. 8C will urge the plunger 870 into engagement against the valve base 850 thus increasing sealing pressure in the valve 800. The increased sealing pressure will reduce the risk of leaking in the event of a sudden surge or spike of fluid pressure in the valve 800, and will guard against an unwanted change of state of the valve 800.

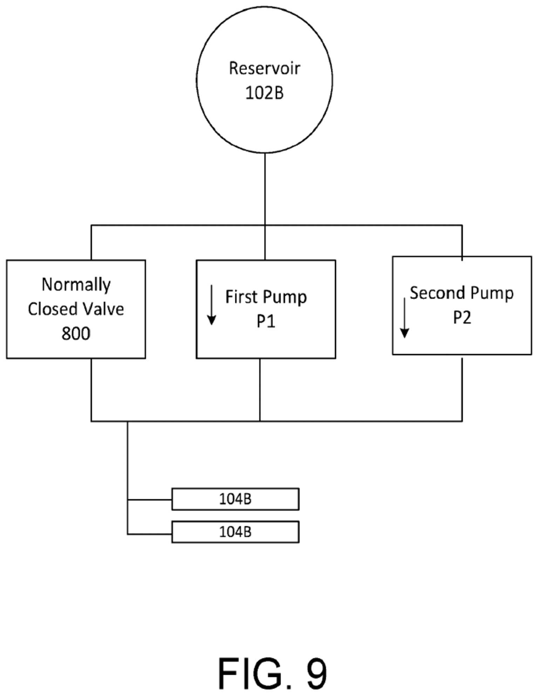

The schematic diagram shown in FIG. 9 illustrates an example fluid architecture for a fluid operated device in the form of the inflatable penile prosthesis 100B described above. In this example arrangement, the inflatable cylinders 104B are in-line with the normally closed valve 800. Thus, when a pressure surge or spike is experienced in the cylinders 104B. the pressure spike could be harnessed to apply the back pressure to the normally closed valve 800 as described above. This may provide for increased sealing pressure in the valve 800 during the pressure spike, thus avoiding leakage of fluid through the valve 800 and maintaining the desired state of the valve 800 and the cylinders 104B.

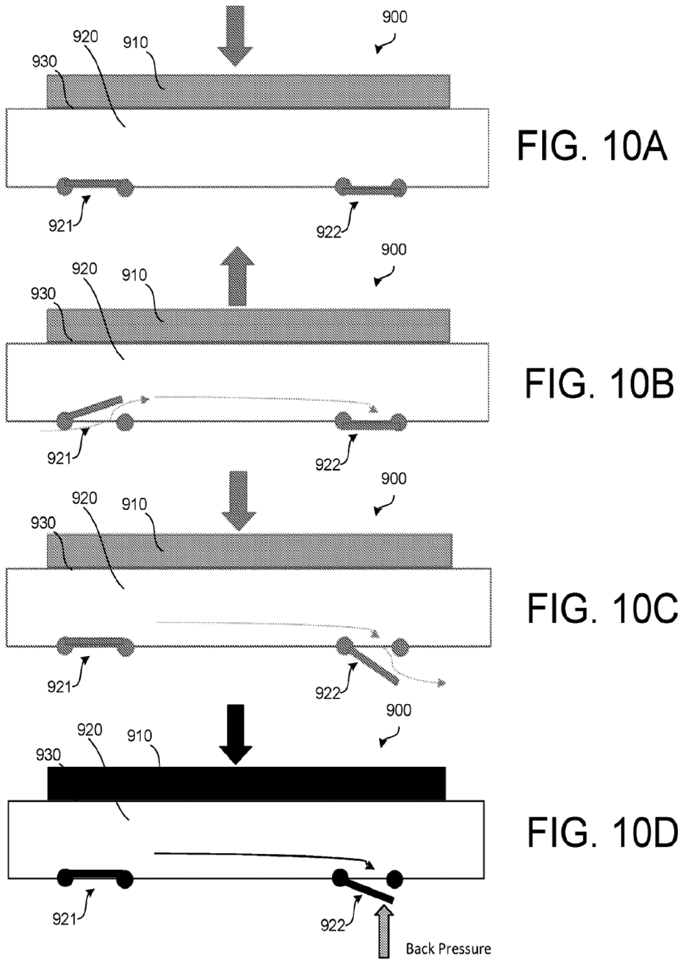

FIGS. 10A-10D illustrate a hybrid, or combined pump and valve device 900 as described above, in which back pressure is applied in response to a detected surge or spike in pressure. The pump and valve device 900 includes a piezoelectric element 910 positioned on a diaphragm 930 along a side of a fluid chamber 920 of the pump and valve device 900. A first check valve 921 is positioned at a first end of the chamber 920, for example, an inlet end of the chamber 920, corresponding to a first end portion of the piezoelectric element 910. The first check valve 921 regulates fluid flow in a first direction, for example, into the chamber 920. A second check valve 922 is positioned at a second end of the chamber 920, for example, an outlet end of the chamber 920, corresponding to a second end portion of the piezoelectric element 910. The second check valve 922 regulates flow in a second direction, for example out of the chamber 920.

A supply stroke, or up stroke, of the pump and valve device 900 (in which the piezoelectric element 910 moves from the concave position shown in FIG. 10A to the convex position shown in FIG. 10B) and the corresponding pressure differential draws fluid into the chamber 920 through the first check valve 921, while the second check valve 922 remains closed. A pressure stroke, or down stroke, of the pump and valve device, including contraction of the piezoelectric element 910, from the convex position shown in FIG. 10B to the concave position shown in FIG. 10C, closes the first check valve 921 and allows fluid to flow out of the chamber 920 through the second check valve 922. The pumping cycle can be repeated to continue to pump fluid into and out of, or through the chamber 920. In this arrangement, application of a backpressure, as shown in FIG. 10D, may provide for forced sealing of the first and second check valves 921, 922 in the event of a spike or surge in fluid pressure that would otherwise result in an unwanted change of state of the fluid operated device.

While certain features of the described implementations have been illustrated as described herein, many modifications, substitutions, changes and equivalents will now occur to those skilled in the art. It is, therefore, to be understood that the appended claims are intended to cover all such modifications and changes as fall within the scope of the embodiments.

The present subject matter includes, inter alia, the following aspects:
1. An implantable fluid operated inflatable device, comprising:
   a fluid reservoir;
   an inflatable member;
   a fluid control system configured to transfer fluid between the fluid reservoir and the inflatable member, including:
      a housing;
      at least one pump positioned in a fluid passageway within in the housing;
      a first fluid port in fluidic communication with the fluid reservoir; and
      a second fluid port in fluidic communication with the inflatable member;
   at least one pressure sensing device configured to sense a fluid pressure in the implantable fluid operated inflatable device; and
   an electronic control system configured to receive the pressure sensed by the at least one pressure sensing device, and to control the at least one valve and at least one pump in response to the received pressure.
2. The implantable fluid operated inflatable device of aspect 1, wherein the at least one pump includes a combined pump and valve device positioned inline between the reservoir and the inflatable member, including:
   a chamber;
   a diaphragm positioned along an edge portion of the chamber;
   a piezoelectric element mounted on the diaphragm;
   a first valve positioned at a first end portion of the chamber corresponding to a first end portion of the piezoelectric element; and
   a second valve positioned at a second end portion of the chamber corresponding to a second end portion of the piezoelectric element.
3. The implantable fluid operated inflatable device of aspect 2, wherein in a first mode in which fluid is moved through the combined pump and valve device in a first direction to transfer fluid from the reservoir to the inflatable member to inflate the inflatable member, a first pumping cycle of the combined pump and valve device includes:
   a first supply stroke in which fluid is drawn into the chamber through the first valve while the second valve is closed; and
   a first pressure stroke in which fluid is expelled out of the chamber through the second valve while the first valve is closed; and
   in a second mode in which fluid is moved through the combined pump and valve device in a second direction to transfer fluid from the inflatable member to the reservoir to deflate the inflatable member, a second pumping cycle of the combined pump and valve device includes:
   a second supply stroke in which fluid is drawn into the chamber through the second valve while the first valve is closed; and
   a second pressure stroke in which fluid is expelled out of the chamber through the first valve while the second valve is closed.
4. The implantable fluid operated inflatable device of aspect 3, wherein
   the first supply stroke and the first pressure stroke are alternately and repeatedly implemented until an inflation pressure of the inflatable member is achieved based on a pressure sensed by the at least one sensing device; and
   the second supply stroke and the second pressure stroke are alternately and repeatedly implemented until a deflation pressure is achieved based on a pressure sensed by the at least one sensing device.
5. The implantable fluid operated inflatable device of any of aspects 1 to 4, wherein the at least one pump is a piezoelectric pump and valve device, including:
   a valve base;
   at least one inlet port formed in the valve base;
   at least one outlet port formed in the valve base;
   a diaphragm coupled to the valve base; and
   a piezoelectric element mounted on the diaphragm,
   wherein a voltage applied to the piezoelectric element is a variable voltage to maintain a set state of the fluid operated inflatable device based on a pressure detected in the fluid passageway of the valve relative to a detected pressure external to the valve.
6. The implantable fluid operated inflatable device of aspect 5, wherein the variable voltage applied to the piezoelectric element to maintain the set state of the fluid operated inflatable device is based on the pressure detected in the fluid passageway of the valve relative to an atmospheric pressure sensed by the electronic control system.
7. The implantable fluid operated inflatable device of aspect 5 or 6, wherein the variable voltage applied to the piezoelectric element adjusts a position of the piezoelectric element and the diaphragm so as to adjust at least one of a fluid pressure or a fluid flow rate to adjust for atmospheric conditions and correspond to the set state of the fluid-controlled inflatable device.
8. The implantable fluid operated inflatable device of any of aspects 5 to 7, wherein the voltage applied to the piezoelectric element is selected from a calibration curve associated with the piezoelectric valve that is accessible in a memory of the electronic control system.
9. The implantable fluid operated inflatable device of any of aspects 5 to 8, wherein the pump and valve device includes a normally open piezoelectric valve that is configured to transition from a normally open state to a closed state in response to an application of voltage to the piezoelectric element, and to return to the normally open state in response to release of the voltage.
10. The implantable fluid operated inflatable device of any of aspects 5 to 9, wherein the normally open piezoelectric valve is configured to remain in the closed state for a period of time after release of the voltage, and to transition to the normally open state in response to dissipation of electrical bias accumulated in the piezoelectric element.
11. The implantable fluid operated inflatable device of any of aspects 5 to 10, the normally open piezoelectric valve further comprising a resistor electrically connected to the piezoelectric element, wherein the resistor is configured to control a dissipation of electrical bias accumulated in the piezoelectric element such that the normally open piezoelectric valve transitions from the closed state to the normally open state in a set period of time after release of the voltage.
12. The implantable fluid operated inflatable device of aspect 5, wherein the piezoelectric valve is a normally closed piezoelectric valve that is configured to transition from a normally closed state to an open state in response to an application of voltage to the piezoelectric element, and to return to the normally closed state in response to release of the voltage, the normally closed piezoelectric valve including:
   a plunger movably positioned within the fluid passageway of the normally closed piezoelectric valve, wherein the plunger is sealed against the valve base in the normally closed state so as to restrict flow through the fluid passageway, and is spaced apart from the valve base in the open state so as to open the fluid passageway.
13. The implantable fluid operated inflatable device of aspect 12, wherein, in the normally closed state of the normally closed piezoelectric valve, a backpressure applied to the plunger through the at least one outlet maintains the sealed position of the plunger against the valve base in response to a surge in fluid pressure at the at least one inlet.
14. The implantable fluid operated inflatable device of any of aspects 1 to 13, wherein the electronic control system includes a printed circuit board including a memory configured to store at least one control algorithm, a communication module configured to communicate with one or more external devices, and a processor configured to:
   receive pressure level measurements from the at least one sensing device;
   apply the at least one control algorithm based on the received pressure level measurements; and
   control operation of the at least one valve and the at least one pump in accordance with the applied at least one control algorithm.
15. The implantable fluid operated device of any of aspects 1 to 14, wherein the implantable fluid operated inflatable device is an artificial urinary sphincter or an inflatable penile prosthesis.
16. An implantable fluid operated inflatable device, comprising:
   a fluid reservoir;
   an inflatable member;
   a fluid control system configured to transfer fluid between the fluid reservoir and the inflatable member, including:
      a housing;
      at least one valve and at least one pump positioned in a fluid passageway within in the housing;
      a first fluid port in fluidic communication with the fluid reservoir; and
      a second fluid port in fluidic communication with the inflatable member;
   at least one pressure sensing device configured to sense a fluid pressure in the implantable fluid operated inflatable device; and
   an electronic control system configured to receive the pressure sensed by the at least one pressure sensing device, and to control the at least one valve and at least one pump in response to the received pressure.
17. The implantable fluid operated inflatable device of aspect 16, wherein the at least one valve and the at least one pump includes a combined pump and valve device positioned inline between the reservoir and the inflatable member, including:
   a chamber;
   a diaphragm positioned along an edge portion of the chamber;
   a piezoelectric element mounted on the diaphragm;
   a first valve positioned at a first end portion of the chamber corresponding to a first end portion of the piezoelectric element; and
   a second valve positioned at a second end portion of the chamber corresponding to a second end portion of the piezoelectric element.
18. The implantable fluid operated inflatable device of aspect 17, wherein
   in a first mode in which fluid is moved through the combined pump and valve device in a first direction to transfer fluid from the reservoir to the inflatable member to inflate the inflatable member, a first pumping cycle of the combined pump and valve device includes:
      a first supply stroke in which fluid is drawn into the chamber through the first valve while the second valve is closed; and
      a first pressure stroke in which fluid is expelled out of the chamber through the second valve while the first valve is closed; and
   in a second mode in which fluid is moved through the combined pump and valve device in a second direction to transfer fluid from the inflatable member to the reservoir to deflate the inflatable member, a second pumping cycle of the combined pump and valve device includes:
      a second supply stroke in which fluid is drawn into the chamber through the second valve while the first valve is closed; and
      a second pressure stroke in which fluid is expelled out of the chamber through the first valve while the second valve is closed.
19. The implantable fluid operated inflatable device of aspect 18, wherein
   the first supply stroke and the first pressure stroke are alternately and repeatedly implemented until an inflation pressure of the inflatable member is achieved based on a pressure sensed by the at least one sensing device; and
   the second supply stroke and the second pressure stroke are alternately and repeatedly implemented until a deflation pressure is achieved based on a pressure sensed by the at least one sensing device.
20. The implantable fluid operated inflatable device of aspect 16, wherein the at least one valve is a piezoelectric valve, including:
   a valve base;
   at least one inlet port formed in the valve base;
   at least one outlet port formed in the valve base;
   a diaphragm coupled to the valve base; and
   a piezoelectric element mounted on the diaphragm,
   wherein a voltage applied to the piezoelectric element is a variable voltage to maintain a set state of the fluid operated inflatable device based on a pressure detected in the fluid passageway of the valve relative to a detected pressure external to the valve.
21. The implantable fluid operated inflatable device of aspect 20, wherein the variable voltage applied to the piezoelectric element to maintain the set state of the fluid operated inflatable device is based on the pressure detected in the fluid passageway of the valve relative to an atmospheric pressure sensed by the electronic control system.
22. The implantable fluid operated inflatable device of aspect 21, wherein the variable voltage applied to the piezoelectric element adjusts a position of the piezoelectric element and the diaphragm so as to adjust at least one of a fluid pressure or a fluid flow rate to adjust for atmospheric conditions and correspond to the set state of the fluid-controlled inflatable device.
23. The implantable fluid operated inflatable device of aspect 20, wherein the voltage applied to the piezoelectric element is selected from a calibration curve associated with the piezoelectric valve that is accessible in a memory of the electronic control system.
24. The implantable fluid operated inflatable device of aspect 20, wherein the at least one valve is a normally open piezoelectric valve that is configured to transition from a normally open state to a closed state in response to an application of voltage to the piezoelectric element, and to return to the normally open state in response to release of the voltage.
25. The implantable fluid operated inflatable device of aspect 24, wherein the normally open piezoelectric valve is configured to remain in the closed state for a period of time after release of the voltage, and to transition to the normally open state in response to dissipation of electrical bias accumulated in the piezoelectric element.
26. The implantable fluid operated inflatable device of aspect 24, the normally open piezoelectric valve further comprising a resistor electrically connected to the piezoelectric element, wherein the resistor is configured to control a dissipation of electrical bias accumulated in the piezoelectric element such that the normally open piezoelectric valve transitions from the closed state to the normally open state in a set period of time after release of the voltage.
27. The implantable fluid operated inflatable device of aspect 20, wherein the piezoelectric valve is a normally closed piezoelectric valve that is configured to transition from a normally closed state to an open state in response to an application of voltage to the piezoelectric element, and to return to the normally closed state in response to release of the voltage, the normally closed piezoelectric valve including:
   a plunger movably positioned within the fluid passageway of the normally closed piezoelectric valve, wherein the plunger is sealed against the valve base in the normally closed state so as to restrict flow through the fluid passageway, and is spaced apart from the valve base in the open state so as to open the fluid passageway.
28. The implantable fluid operated inflatable device of aspect 27, wherein, in the normally closed state of the normally closed piezoelectric valve, a backpressure applied to the plunger through the at least one outlet maintains the sealed position of the plunger against the valve base in response to a surge in fluid pressure at the at least one inlet.
29. The implantable fluid operated inflatable device of aspect 16, wherein the electronic control system includes a printed circuit board including a memory configured to store at least one control algorithm, a communication module configured to communicate with one or more external devices, and a processor configured to:
   receive pressure level measurements from the at least one sensing device;
   apply the at least one control algorithm based on the received pressure level measurements; and
   control operation of the at least one valve and the at least one pump in accordance with the applied at least one control algorithm.
30. The implantable fluid operated device of aspect 16, wherein the implantable fluid operated inflatable device is an artificial urinary sphincter or an inflatable penile prosthesis.
31. A method of controlling an implantable fluid operated inflatable device, comprising:
   receiving, by a processor of the inflatable device from a pressure sensing device within a fluid passageway of the inflatable device, a fluid pressure measurement;
   comparing, by the processor, the measured pressure received from the pressure sensing device to a pressure external to the fluid passageway; and
   controlling, by the processor, a circuit to apply a voltage to a piezoelectric element of a piezoelectric valve of the inflatable device based on the comparison to maintain a set condition of the inflatable device.
32. The method of aspect 31, wherein controlling the circuit to apply the voltage to the piezoelectric element includes:
   detecting, based on the comparison, a change in atmospheric pressure from a calibration condition of the inflatable device based on the comparison;
   selecting, by the processor, a voltage to be applied to a piezoelectric element of a piezoelectric valve of the inflatable device from a previously stored lookup table in response to the detected change in atmospheric pressure; and
   applying to the selected voltage to the piezoelectric element to maintain a set condition of the inflatable device in the changed atmospheric conditions.
33. The method of aspect 31, wherein the piezoelectric valve is a normally open piezoelectric valve, and wherein controlling the circuit to apply the voltage to the piezoelectric element includes controlling a resistor in the circuit such that electrical bias accumulated in the piezoelectric element dissipates over a set period of time to return the normally open piezoelectric valve to a normally open state.
34. The method of aspect 31, wherein the piezoelectric valve is a normally closed piezoelectric valve, the method further comprising:
   detecting a surge in fluid pressure at an inlet portion of the piezoelectric valve; and
   applying a backpressure at an outlet portion of the piezoelectric valve in response to the surge in fluid pressure at the inlet portion to maintain a closed state of the normally closed piezoelectric valve.
35. The method of aspect 31, further comprising:
   receiving, by a control module of the processor, a user input from an external device in communication with the processor; and
   adjusting at least one of a fluid pressure or a fluid flow rate in the inflatable device in response to the received user input.

## Claims

1. An implantable fluid operated inflatable device (100), comprising:
a fluid reservoir (102);
an inflatable member (104);
a fluid control system (106) configured to transfer fluid between the fluid reservoir (102) and the inflatable member (104), including:
a housing (110A, 110B);
at least one valve (V1, V2) and at least one pump (P1, P2) positioned in a fluid passageway within in the housing (110A, 110B);
a first fluid port (107A, 107B) in fluidic communication with the fluid reservoir (102); and
a second fluid port (109A, 109B) in fluidic communication with the inflatable member (104);
at least one pressure sensing device (S1, S2) configured to sense a fluid pressure in the implantable fluid operated inflatable device (100); and
an electronic control system (108) configured to receive the pressure sensed by the at least one pressure sensing device (S1, S2), and to control the at least one valve (V1, V2) and at least one pump (P1, P2) in response to the received pressure,
wherein the at least one valve (V1, V2) is a piezoelectric valve (400) that is configured to transition from an open state to a closed state in response to an application of voltage to a piezoelectric element (410), and to return to the open state in response to release of the voltage.

2. The implantable fluid operated inflatable device (100) of claim 1,
wherein the piezoelectric valve (400) further includes:
a fluid chamber (420),
a first fluid port (415),
a second fluid port (420), and
a valve base (450),
wherein the piezoelectric element (410) is coupled to the valve base (450), the fluid ports (415, 425) are formed in the valve base (450), the first fluid port (415) is an inlet port, the second fluid port (425) is an outlet port, and the fluid chamber (420) is defined in a space between the piezoelectric element (410) and the valve base (450).

3. The implantable fluid operated inflatable device (100) of claim 1 or 2,
wherein the piezoelectric valve (400) further includes a diaphragm.

4. The implantable fluid operated inflatable device (100) of any one of claims 1 to 3,
wherein the piezoelectric valve (400) is configured to remain in the closed state for a period of time after release of the voltage, and to transition to the open state in response to dissipation of electrical bias accumulated in the piezoelectric element (410).

5. The implantable fluid operated inflatable device (100) of any one of claims 1 to 4, wherein the piezoelectric valve (400) includes a resistor (490),
wherein the resistor (490) is configured to control a dissipation of electrical bias accumulated in the piezoelectric element (410) such that the piezoelectric valve (400) transitions from the closed state to the open state in a set period of time after release of the voltage

6. The implantable fluid operated inflatable device (100) of any one of claims 1 to 5,
wherein the voltage level applied to the valve (400) can ba varied to vary an opening amount of the valve (400) or to vary a flow rate through the valve (400).

7. The implantable fluid operated inflatable device (100) of claim 6,
wherein variable voltage control can be applied to account for changes in atmospheric pressure which affect pressure of fluid flowing in the fluid operated device (100), and thus can affect the proper operation of the fluid operated device (100).

8. The implantable fluid operated inflatable device (100) of any one of claims 1 to 7,
wherein the implantable fluid operated inflatable device (100) is configured to obtain atmospheric and/or working pressure substantially real time.

9. The implantable fluid operated inflatable device (100) of claim 8,
wherein the electronically controlled fluid operated device (100) uses detected atmospheric and/or working pressure levels, and detected changes in atmospheric and/or working pressure levels, to adjust pressure levels in the fluid operated device (100), and in particular to adjust operation of flow control valves in the fluid operated device (100) to ensure proper fluid pressure levels and fluid flow rates for safe operation of the fluid operated device (100).

10. The implantable fluid operated inflatable device (100) of any one of claims 1 to 9,
wherein a variable closing voltage is applied to one or more of the valves (400) in the fluid operated device (100) to provide a corrected level of closure of the valve (400) corresponding to varying levels of atmospheric and/or working pressure the valve (400) experiences.

11. The implantable fluid operated inflatable device (100) of any one of claims 1 to 10,
wherein a calibration curve is referenced to determine an appropriate closing voltage for a given atmospheric and/or working pressure sensed by the electronically controlled fluid operated device (100).

12. The implantable fluid operated inflatable device (100) of any one of claims 1 to 11,
wherein a calibration curve is stored in the form of a look up table, in a memory of the electronic control system (108).

13. The implantable fluid operated inflatable device (100) of any of claims 1 to 12,
wherein the electronic control system (108) includes a printed circuit board including a memory configured to store at least one control algorithm, a communication module configured to communicate with one or more external devices, and a processor configured to:
receive pressure level measurements from the at least one sensing device;
apply the at least one control algorithm based on the received pressure level measurements; and
control operation of the at least one valve and the at least one pump in accordance with the applied at least one control algorithm.

14. The implantable fluid operated device (100) of any of claims 1 to 13,
wherein the implantable fluid operated inflatable device (100) is an artificial urinary sphincter (100A) or an inflatable penile prosthesis (100B).

15. The implantable fluid operated device (100) of any of claims 1 to 14,
wherein a valve (V1, V2) and a pump (P 1, P2) are incorporated into a single component that provides for both the generation of fluid flow, and the control of fluid flow in the fluid operated device (100).
